# EUROPEAN PATENT APPLICATION

(11) **EP 4 571 764 A1**
(43) Date of publication of application: **18.06.2025**
(21) Application number: 23217153.8
(22) Date of filing: 15.12.2023
(51) Int. Cl.: G16H 30/20, G06N 3/045, G06N 3/08, G09B 5/02, G16H 30/40, G16H 50/50

(54) **METHOD FOR CREATING A SYNTHETIC PATIENT MODEL**

(71) Applicant: Koninklijke Philips N.V., 5656 AG Eindhoven (NL)
(72) Inventor: KOLOKOLNIKOV, Georgii, Eindhoven (NL); WISSEL, Tobias, Eindhoven (NL); BARAGONA, Marco, 5656AG Eindhoven (NL); BAKKER, Bart Jacob, Eindhoven (NL); BUERGER, Christian, Eindhoven (NL)
(74) Representative: Philips Intellectual Property & Standards

(57) **Abstract**

Provided is a computer-implemented method for creating a synthetic patient model comprising a synthetic patient image. The method comprises: obtaining a data set comprising medical information, wherein the medical information comprises an anatomical description comprising at least information specifying an anatomical structure to be depicted in the synthetic patient image, and imaging information specifying image formation characteristics of the synthetic patient image, and wherein the medical information optionally comprises anamnesis and physical examination descriptions; generating an anatomical map of the anatomical structure by means of anatomy modelling using the anatomical description as an input; conditioning a generative model using the anatomical map; embedding a subset of the medical information, the subset comprising textual information and including at least the imaging information, in the generative model; creating the synthetic patient model including a synthetic patient image by means of the generative model.

## Description

### FIELD OF THE INVENTION

The invention provides computer-implemented method for creating a synthetic patient model, a system, a computer program product, and a computer readable medium.

### BACKGROUND OF THE INVENTION

At present, there are limits when it comes to train and/or support practitioners or ML models for accurate analysis of medical images acquired for a specific patient. One of the reasons is that the number of available data sets is rather small and may not be representative of all potential scenarios, e.g., lack granularity. Moreover, even for available data sets, it may be very difficult to retrieve data sets, e.g. reference images that are pertinent for the case at hand.

This can cause problems in various areas of analysis of medical images, such as X-ray image or CT images, which in the end may cause inadequate accuracy and reliability.

For example, available data sets may not be suitable for accurately training a machine learning model to analyze medical images reliably. Similarly, providing adequate support systems for training medical professionals or students for analyzing medical images or supporting systems for medical professionals in analyzing medical images may not be possible.

All of the above may lead to inadequate reliability or accuracy when analyzing medical images.

It is an object of the invention to provide a method and system that allows for improving reliability and/or accuracy when analyzing medical images.

### SUMMARY OF THE INVENTION

The invention provides a computer-implemented method for creating a synthetic patient model, a system, a computer-readable medium and a computer program product according to the independent claims. Preferred embodiments are laid down in the dependent claims.

The present disclosure provides a computer-implemented method for creating a synthetic patient model comprising a synthetic patient image. The method comprises:
obtaining a data set comprising medical information, wherein the medical information comprises an anatomical description comprising at least information specifying an anatomical structure to be depicted in the synthetic patient image, and imaging information specifying image formation characteristics of the synthetic patient image, and wherein the medical information optionally comprises anamnesis and physical examination descriptions;
generating an anatomical map of the anatomical structure by means of anatomy modelling using the anatomical description as an input;
conditioning a generative model using the anatomical map;
embedding a subset of the medical information, the subset comprising textual information and including at least the imaging information, particularly the imaging information being or comprising textual information, in the generative model; and
creating the synthetic patient model including a synthetic patient image by means of the generative model.

Using a generative model for creating synthetic data, such as synthetic images, in general, can yield results with varying efficiency and quality. In the present case, rather than indiscriminately inputting medical information into the generative model, the method branches off and (directly) embeds part of the medical information in the model, and part of the information is used to generate an anatomical map, which is then used for conditioning the model. This allows for yielding high quality and efficiency, because the anatomical map can impose clear spatial constraints and potentially also other constraints that guide the model generation.

It will be understood from the above that by using a generative model and two branches, i.e., a branch that embeds a subset of the medical information as such and a branch that uses a subset of the medical information to create an anatomical map, it is possible to create meaningful synthetic patient images as part of a synthetic patient model in a reliable manner and at an effort that allows for creating also larger data sets having synthetic patient models for a large number of inputs. The synthetic patient images can be used in various ways to improve reliability and/or accuracy when analyzing medical images, as they allow for training ML models or medical professionals or

In the present disclosure, the term "patient model" is to be understood as a data set that is representative of a patient, i.e., models a patient. A patient model may comprise different categories or types of information, such as image information, textual information, signals, or the like. The term "synthetic patient model", in the present disclosure, may be understood as a patient model that comprises synthetized information. Synthesized information may, for example, be obtained by modelling. Optionally, in addition to synthesized information, the synthetic patient model may also comprise non-synthesized information, such as image data acquired by an imaging device, and/or may also comprise information extracted from non-synthesized information, such as image data. A synthetic patient image, according to the present disclosure, may be an image yielded by a modelling, particularly by the generative model. The synthetic patient image may be an image that imitates images of a patient, e.g., captured by a specific imaging modality. For example, the synthetic patient image may be an image that imitates an X-ray image. The synthetic patient image may, for example, be a synthetic medical image, such as synthetic X-ray, CT, or MRI image.

The patient referred to herein may be an artificial construct. The patient may be characterized by patient characteristics included in the medical information. However, it is not excluded that at least part of the medical information is representative of a real patient. As an example, the medical information may be representative of a real patient and may then be modified, e.g., to simulate how progression of a medical condition and/or a medical intervention would manifest in a medical image of the patient.

The medical information, as will be discussed in more detail below, may comprise different types of information, for example textual information and/or image information. The medical information may comprise medical descriptions, for example natural language descriptions. In particular, the medical information may be structured information. That is, the information may be a predetermined structure. Information may be brought into the predetermined structure by appropriate data input and/or by processing input data, such as by a language model and/or parsing the input data.

While structured data may improve efficiency and/or accuracy, unstructured data may also be usable.

The anatomical description comprised in the medical information may, for example, be representative of an anatomy to be represented in the synthetic patient model, particularly to be depicted in the synthetic patient image. Details will be provided further below. As such, the medical information may specify which anatomical structure the synthetic patient image will represent. Using other parts of the medical information allows to further specify what the synthetic patient image will represent, as is explained in the following.

The medical information also comprises imaging information specifying image formation characteristics of the synthetic patient image. Image formation characteristics may comprise an imaging modality and/or imaging parameters, such as imaging settings of an imaging device, or the like. The synthetic patient image, thus, may not only have appropriate geometry, but also be generated with the characteristics of a specified imaging procedure. As an example, for the same anatomical description, e.g. the same anatomical structure to be modelled, synthetic images representative of different imaging modalities may be created depending on the image formation characteristics.

The medical information optionally comprises anamnesis and physical examination descriptions. For example, this may comprise a description including age, weight and gender of the patient or a description of a reason for a patient to present to a physician, e.g., indicating that a patient presented himself with shortage of breath (dyspnoe) under stress and chest pain or quickly experienced fatigue and muscle pain under physical exercise. In other examples, patient history of therapy, medication and/or disease may play a role. E.g., a patient may present with a history of a number of years of diabetes and peripheral artery disease, may have received a coronary stent in proximal Left Anterior Descending (LAD) artery or was suffering from left heart insufficiency and valve calcification and was implanted an artificial aortic valve during a previous TAVI procedure. In other examples, medical history may list a lobectomy that had taken place before. As another example, current diagnoses and associated information, such as diagnostic imaging may be included in the medical information.

The term "obtaining medical information" may comprise generating medical information by processing information from one or more data sources, for example information retrieved from storage and/or received directly and/or indirectly from a user input. This may be the case where a user input is used as a prompt for a language model and the medical information is based on the output of the language model and/or where received/retrieved information is parsed, and the medical information comprises the parsed information. Alternatively or in addition, information may also be included as is, e.g., retrieved and/or received, in the medical information, e.g. without prior processing.

The method comprises generating an anatomical map of the anatomical structure by means of anatomy modelling using the anatomical description as an input. An anatomical map is a map of an anatomical structure. It may provide spatial information about the anatomical structure. Particularly, the anatomical map may be a semantic anatomical map.

The semantic anatomical map may comprise an anatomical map, wherein each spatial element in the map (e.g. 2D pixel or 3D voxel) is assigned to at least one out of a discrete set of anatomical classes, for example organ type or tissue characteristics. The assignment may be represented by labels, e.g. semantic labels, in which case the semantic anatomical map may be considered to be a labelled map, particularly having semantic labels. Alternatively or in addition, the semantic anatomical map may also contain continuous indicators (e.g. of floating point numbers between 0 and 1) or a vector thereof per element that characterize this element anatomically, for example in form of an anatomical class probability vector that in each element indicates how likely it is that an element belongs to each of a set of possible anatomical classes. This may be helpful at semantic transition zones where e.g. spatial elements may belong to not only one but several anatomical classes.

Conditioning of the generative model and embedding the input information will be described in more detail below.

The subset of medical information to be embedded (i.e., embedded into another representation for subsequent processing steps within the generative model) may comprise at least the imaging information, particularly the image formation characteristics, such as modality and/or imaging parameters. Thus, after conditioning the generative model with the anatomical map and the imaging information, the generation will be constrained in a suitable manner to yield high quality synthetic images in accordance with the medical information.

According to the present disclosure, a synthetic patient model including a synthetic patient image is created by means of the generative model. That is, the generative model yields output, particularly yields the synthetic patient image as an output, and said output is included in the synthetic patient model. Optionally, the synthetic patient model may additionally comprise other information, such as from intermediate processing steps and/or portions of the medical information and/or information yielded by other models and/or non-synthetic information.

According to the present disclosure, conditioning the generative model using the anatomical map may comprise conditioning the generative model directly with the anatomical map or generating an embedding of the anatomical map and conditioning the generative model with the embedding of the anatomical map.

According to the present disclosure, generating the anatomical map may comprise obtaining spatial information of the anatomical structure by means of the anatomy modelling and generating a labelled mesh representation of the surface of the anatomical structure or a label mask of the anatomical structure. Obtaining spatial information of anatomical structures and creating mesh representations or masks of anatomical structures can make use of known, e.g. atlas-based, anatomy modelling techniques. Generating labelled mesh representations and/or a label mask can also make use of known techniques. The anatomical map may be generated fully automatically (including the labels).

The anatomical maps specified above are advantageous as input for a generative model in the context of the present disclosure, that is, as specified above and in the claims. Other anatomical maps may also optionally be used, but the anatomical maps, particularly semantic anatomical maps, yield particularly good results when used in this context.

According to the present disclosure, the anatomical description may comprise one or more organs to be modelled, such as heart, lung, liver, kidney. As an example, the anatomical description may comprise a textual description. Herein, a textual description may refer to alphanumerical description, for example. The organs may be specified by their name(s) and/or by an identifier, which may be selected by a user as part of a user input and/or may be derived from a user input. The identifier may be included in the medical information instead of or in addition to a textual description. Thus, a user may, for example, specify an organ at least part of which is to be depicted on the synthetic patient image. Anatomy modelling may use this specification for creating an anatomical map of the organ and optionally its surroundings. As the anatomical map is used by the generative model that generates the synthetic patient image based thereon, by specifying the organ to be modelled in the medical information, it can be specified which organ is to be depicted in the synthetic patient image.

Alternatively or in addition, the anatomical description may comprise one or more shape descriptors for the anatomical structure, particularly general shape descriptors and/or pathological shape descriptors. The shape descriptors may optionally comprise at least one of: pathological conditions, particularly pathological conditions that influence anatomy, a pathology-specific shape and/or deformation of an anatomical structure, such as pathology-specific deformations of an anatomical structure. Alternatively or in addition, the shape descriptors may optionally comprise a physiological state of an organ to be modelled, such as cardiac phase, e.g. end systole (ES) or end diastole (ED), or respiratory phase, such as inhale or exhale phase of the lung.

As non-limiting examples, the pathological shape descriptors may comprise, for the heart as an exemplary anatomical structure, a cardiomyopathy type characterizing hypertrophy (thickened myocardium), apical hypoplasia, truncated, enlarged, elongated, spherical chambers (e.g., left atrium, LA; left ventricle, LV; right atrium, RA; right ventricle, RV), one chamber wrapped around the other, chamber dilation, "banana-shaped" RV etc. Other pathological conditions may also influence the anatomy, such as calcified valves.

Alternatively or in addition, the shape descriptors may optionally comprise a location and/or characteristic of a lesion to be modelled. For example, they may specify the organ and optionally a specific region of an organ where a lesion is located. Alternatively or in addition, the type and/or size and/or shape of the lesion may be specified.

Taking into account shape descriptors allows for more a higher accuracy anatomic model and, as a consequence, a higher accuracy synthetic patient model.

Shape descriptors may, particularly, comprise an alphanumerical representation of the shape descriptor, e.g. a word and/or an ID.

Alternatively or in addition, the anatomical description may comprise a previous medical intervention, such as a heart valve clip, a prosthesis, and/or a stent. Thus, for example, the output of the generative model, particularly the synthetic patient image, may model effects of the previous medical intervention. For example, an object, such as a prosthesis, or anatomical changes brought about by the previous medical intervention might be depicted in the synthetic patient image, for example in the manner it would be observed with the selected imaging modality and/or rendered as an overlay, e.g. in case it would not be observed with the selected imaging modality. Thus, better accuracy can be provided.

Alternatively or in addition, the anatomical description may comprise patient characteristics such as age, size, weight, BMI, ejection fraction for the heart. Such information may, when input into an anatomy modelling, yield different sizes and/or shapes of anatomical features in the anatomical model and, subsequently, in the synthetic patient image generated based thereon. Accordingly, better overall accuracy can be achieved by providing the characteristics.

According to the present disclosure, the anatomical description may be in the form of textual data and/or image data. Where the anatomical description is in the form of image data, during or prior to further use by the anatomy modelling and/or the generative model, the image data may be processed to extract information to be used in the further processing. As an example, parts of structures in image data may be segmented and the segmented data may be used during the (knowledge-based) anatomy modelling. For example, in case a pre-existing deformation of an anatomical structure is depicted in the image data, this may thus be taken into account in the anatomy modelling, particularly even if it is textually described in the medical information. As another example, if the image data comprises a sequence of ultrasound images of a beating heart, anatomical characteristics, such as contraction or torsion, of the systolic phases can be extracted and used in generating the synthetic patient image. In case the subset of medical information embedded in the generative model asks for a specific systolic phase, such as a generation of a CTA image in end-systole phase, the generative model may yield a synthetic image representative of the said phase directly or indirectly (via the anatomy modelling) making use of the extracted characteristics. Alternatively or in addition, patient specific aspects may - based on the anatomical description - be classified into a set of discrete options, such as a collection of pre-defined heart shapes or thicknesses of LV myocardium. In one example and based on such a classification one of several pre-defined anatomy templates or statistical modes can be chosen or modified.

According to the present disclosure, anatomy modelling may comprise generating a surface model of the anatomical structure's surface using a surface mesh or generating a volumetric model of the anatomical structure. Generating the anatomical map may comprise generating labels, such as semantic labels, for one or more portions of the surface model or the volumetric model, particularly labels associating information with the one or more portions. The one or more portions may comprise at least one of: surface portions, volumetric portions, locations. In particular, the modelling may be a knowledge-based modelling making use of anatomical atlas data and optionally spatial data representative of artificial structures, such as prosthesis, heart valve clips, stents or the like. For anatomy and for artificial structures, there is general knowledge that is used as a basis for the anatomy modelling. Therefore, the anatomy modelling may be considered knowledge-based modelling.

The above allows for obtaining a surface model that accurately represents an anatomical structure to be depicted in the synthetic patient model. In general, known methods for anatomy modelling, particularly methods that yield (semantic) anatomical models, may be used. Particularly, known methods the use of atlas data for modelling may be used.

According to the present disclosure, obtaining the data set comprising medical information may comprise parsing information, for example, parsing textual information, and extracting, from the parsed information, information to be input into the anatomy modelling and/or to be embedded in the generative model. This may, for example, improve accuracy and/or efficiency of the subsequent data processing or, in some cases be necessary for subsequent steps in case they require information to be input in a very specific form.

Parsing information, for example parsing information input by a user, may comprise breaking down and/or separating input information, such as textual information, analyzing the information in terms of structure and components, and converting the information into a structured format. Parsing may also comprise condensing information The structured format may be predetermined and, for example, selected to yield suitable input for the anatomy modelling and/or for the embedding it in the generative model. Parsing methods are known in the art and the skilled person would select a parsing method according to the application at hand.

The medical information may comprise parsed and/or unparsed information. Accordingly, parsing the information is an optional step.

For example, the generative model and/or the anatomy modelling (knowledge-based modelling) may be configured to ingest information that has not or only partially been parsed, in which case parsing of information may completely or partially omitted.

According to the present disclosure, obtaining the data set comprising medical information may comprise receiving a user input specifying information to be included in the medical information and/or receiving user input from which information to be included in the medical information is derived, and including the specified and/or derived information in the medical information. User input or information derived from the user input may, for example, specify at least one of patient characteristics, like sex, age, height, weight, an imaging modality for the synthetic image, a patient anatomy to be imaged, or the like. A user input may be received via a user interface allowing for user interaction, for example using a touch display, a mouse, a keyboard, or a combination thereof. Deriving information from the user input may, for example, comprise generating information based thereon, e.g. by using it as a prompt for a generative model, and/or otherwise processing the user input, e.g. by extracting information from the user input.

According to the present disclosure, at least part of the medical information may be textual information. As an example, textual information may comprise textual information specifying patient characteristics like sex, age, height, weight, and/or specifying an imaging modality for the synthetic image, specify an anatomy to be imaged, and/or specifying a medical condition, or the like.

Alternatively or in addition, the medical information may comprise image data and/or signal data, such as medical images and/or EEG and/or ECG signals.

According to the present disclosure, the medical information may comprise information in the form of a medical report. Medical reports often have a pre-defined structure and/or pre-defined categories of content. As such, information in medical reports may be more structured than free-form information, which may improve efficiency and/or quality of data processing.

According to the present disclosure, the user input may comprise a selection of information and the selected information may be included in the medical information.

As an example, a user may select from one or more lists, e.g., visualized by drop-down menus, information to be included in the medical information. As an example, user may select a medical condition, and/or patient characteristics like sex, age, height, weight, and/or an imaging modality for the synthetic image, or the like. An advantage is that such information is well-structured and the input values may be limited and controlled. As such, it may avoid input data complexity where it is not required and improve processing quality and efficiency.

According to the present disclosure, the user input may comprise formalized free input, for example a user filling in a digital form, and the method may comprise extracting the medical information by processing the formalized free input. This type of input may still yield we-structured input, with corresponding advantages in terms of quality and efficiency, but it may also help accounting for more individualized inputs that cannot easily be reflected in a selection list. This type of input may be combined with a selection of information as described above. For example, some information may be selected from a list while other information may be typed into a form.

According to the present disclosure, the user input may comprise one or more prompts for a language model, particularly a large language model such as a Generative Pre-trained Transformer, GPT,-based model, and the method may comprise providing the one or more prompt to the language model and the language model outputting the medical information by processing the one or more prompts. This may be used alone or in combination with the above-described selection and/or formalized free input.

Such a user input allows for high flexibility in terms of which information is provided and how it is described. This high flexibility allows for accounting for very complex scenarios or for use by inexperienced users that may not have the knowledge allowing for a more formalized input. Moreover, it may, in some cases, make the input process more efficient, as the user can quickly input a large amount of information. This may make such an input advantageous in terms of overall efficiency, even if the data processing process may be a bit more computationally expensive than for more formalized input. It is noted that language models are very adept at yielding structured output from rather unstructured input. Structured output may yield improved results from subsequent processes, such as the anatomy modelling, which may expect a rather specific type of input, and/or the generative model that yields the synthetic medical report.

According to the present disclosure, the embedding of the subset of the medical information in the generative model may comprise transforming the subset of medical information into an embedding space, particularly comprising tokenization of textual information comprised in the subset of the medical information and generating a condensed representation by transforming the tokens with a, particularly given or learned, transformation. This yields data that is particularly suited for being ingested by the generative model, particularly allowing for high quality output and/or high efficiency. Particularly, such an embedding is well-suited for processing textual information comprised in the medical information by means of the generative model.

The method may further comprise, as part of creating the synthetic patient model, generating synthetic 1D data, such as signals like ECG or EEG signals, by means of a text-to-signal generative model conditioned on one or more text embeddings of textual information comprised in the medical information.

As an example, the medical information may comprise a textual description of past and/or present heart and/or neurological conditions. Such descriptions may be used as input for a text-to-signal generative model conditioned on text-embeddings of the textual description. The text-to-signal generative model may be trained to generate an ECG and/or EEG signal based on the textual description, for example including irregularities that may be associated with the heart and/or neurological conditions. As an example, this may allow for generating a set of training data for an ML model to train it to predict from a medical image a 1D signal and/or vice versa, e.g. predicting from a medical image depicting heart and/or brain damage, ECG and/or EEG signals.

According to the present disclosure, the generative model may be a machine learning model, particularly a diffusion model, particularly a latent diffusion model.

Diffusion models, also known as diffusion probabilistic or score-based generative models, are a class of generative models, examples of which are known in the art. In principle, they generate data by reversing a diffusion process, where a diffusion process gradually adds noise to data. The model is trained to reverse the diffusion process, i.e. to start from and gradually remove noise to generate output.

Particularly, in the present disclosure, a latent diffusion model may be employed. Examples for latent diffusion models are known in the art. Latent diffusion models are particularly advantageous where a generative model generates image output (image generation), while also being able to handle other types of data, and particularly, also being suitable for use in multimodal scenarios. Latent diffusion models may particularly rely on the aforementioned embedding mechanism to efficiently handle memory-intense image information (e.g. where parts of the image space representation are redundant pieces of information and can be compressed).

The above examples for generative models, in the context of the present disclosure, yield good quality and efficiency. However, other generative models may also be used, which may, depending on the application, differ in efficiency and/or quality.

According to the present disclosure, the synthetic patient model may comprise a synthetic medical report, particularly comprising at least part of the medical information, and/or an output of the anatomy modelling, such as the anatomical map (knowledge-based output, e.g. ground truth annotations), and/or a/the synthetic 1D data.

The synthetic medical report may be a report that comprises text and/or image data. The synthetic may comprise the synthetic patient image. The synthetic medical report may comprise at least part of the medical information and/or information from which the medical information was derived and/or information extracted from the medical information. The synthetic medical report may comprise intermediate outputs of the method of the present disclosure, such as output of the anatomy modelling, e.g., the generated anatomical map. The synthetic medical report may optionally comprise non-synthetic information, such as real medical images, e.g. retrieved from a data storage. The synthetic medical report may be generated fully automatically and/or based on a user input. The generating of the synthetic medical report may comprise defining a configuration of the synthetic medical report, such as configuration of which type of information to be included in the synthetic medical report. As an example, a user or a pre-configured filter may select to include, in addition to the synthetic patient image, additional information, such as at least part of the information that was input into the generative model or other synthetic information like a synthetic signal, to the synthetic medical report. Adding more information to the synthetic medical report may allow for improved usage of the synthetic medical report, e.g., where another model ingests and processes the synthetic medical report and, for example, may make use of the at least part of the additional information, such as information that was input into the generative model. This may also be used for generating a suitable synthetic patient image for training a ML model on the synthetic medical report. Thus, configuring the synthetic medical report may allow for creating a synthetic medical report according to the technical requirements for further using the synthetic medical report.

The method of the present disclosure may also comprise providing one or more tools based on one or more synthetic patient models obtained by any of the methods described above.

The one or more tools may comprise an analysis support tool for supporting image analysis by a medical practitioner. As an example, a medical practitioner may want to analyze a medical image obtained for a patient. The analysis support tool may provide functionality that allows for the medical practitioner to generate one or more synthetic patient models based on input information that characterizes the patient at hand to aid in analyzing certain aspects of the medical image. As an example, the medical practitioner may be aware, for example from medical imaging using a first imaging modality, that a patient has a certain condition, such as a tumor in a specific region of an organ. The medical practitioner may want to create a synthetic patient model to understand how, in this specific patient, this condition, e.g. tumor, would show up in medical images acquired by a second imaging modality, different from the first imaging modality. A synthetic patient model modelling the second imaging modality may be generated and used for supporting analyzing images acquired with the second imaging modality. For example, the synthetic patient model may allow for a medical practitioner to be able to distinguish how the tumor would be expected to appear in the second imaging modality for this specific patient and, thus, may more accurately analyze the medical image acquired with the second imaging modality. As a consequence, for example, this may increase precision of image analysis and/or allow for monitoring changes of the tumor with the second imaging modality, which may be advantageous where use of the first imaging modality is or should be limited for resource or patient protection reasons.

Alternatively or in addition, the one or more tools may comprise a training tool for training of a student and/or medical practitioner.

The training tool may allow for a student and/or a medical practitioner to observe how changes in the input data relating to patient, such as size, age, or the like, and/or relating to a medical condition, would manifest in a medical imaging modality. Training can thus be carried out according to need, for example, specialized for children or specialized to certain part of the body depending on a field of endeavor of the medical practitioner, and with high granularity that will likely not be available from existing image data and/or based on data that may be difficult to find and/or access, e.g., distributed over various publications and potentially only in paper-form.

Alternatively or in addition, the one or more tools may comprise a training data generation tool configured to generate training data for a machine learning model, the training data based on and/or derived from the one or more synthetic patient models. The training data generation tool may be configured to automatically or semi-automatically generate a plurality of synthetic patient models. The plurality of synthetic patient models or subset thereof may be used as training data for a machine learning model. Accuracy of a trained machine learning model depends on the quality of the training data. The amount of training data cannot be too low, and the training data should not cover only few scenarios, but preferably cover a wide range of scenarios. As explained above, both can be difficult to achieve for medical imaging. Moreover, having accurately labelled data is advantageous for many machine learning approaches, yet labelling actual image data can be quite cumbersome. The training data generation toll may overcome these challenges and allow for an accurately trained machined learning model. A machine learning models may, for example, be trained to ingest medical images acquired with a medical imaging modality and yield analysis of the medical images.

The present disclosure may provide use of the analysis support tool for analysis support for a medical practitioner, the analysis support tool assisting the medical practitioner in analyzing medical images. Reference is made to the description of the analysis support tool provided above.

The present disclosure may provide use of the training tool for training medical practitioners and/or students, particularly to train their medical image analysis accuracy. Reference is made to the training tool provided above.

The present disclosure may provide use of the training data generation tool for generating training data. Furthermore, the present disclosure may provide use of the generated training data for training a machine learning model, particularly a machine learning model trained to ingest medical images acquired with a medical imaging modality and yield analysis of the medical images. Reference is made to the description of the training data generation tool provided above.

The present disclosure also provides a system comprising one or more processing devices configured to carry out the method of the present disclosure.

The present disclosure also provides a computer program product comprising computer-readable instructions which, when executed by a computer, cause the computer to carry out the method of the present disclosure, particularly as outlined above and as claimed.

The present disclosure also provides a computer-readable medium having stored thereon computer-readable instructions which, when executed by a computer, cause the computer to carry out the method of the present disclosure, particularly as outlined above and as claimed.

Below, some general information on how embedding and conditioning may be understood according to the present disclosure are provided. However, it is noted that the concepts of embedding and conditioning are known to the skilled person and the below merely serve as non-limiting examples.

Embedding: Embedding may refer to taking a piece of information in one representation and transforming it into another representation which is more advantageous for some purpose (e.g. more compressed). The information processed by the model may be provided in the form of medical text. In order to carry out a number of processing steps on this information, the text may be converted into a numerical representation that compresses, but most importantly preserves the target information encoded in the text. To achieve this, the text may be split into fragments. These fragments are called tokens (e.g., utf-8 unicode representation of letters or combination of letters) and the process is referred to as tokenization. The resulting stream of tokens may then be transformed into even more condensed representations by a learned or given transformation (e.g. a linear projection). These representations are called embeddings, as they embed the original text information into a different space, which can be seen as a more appropriate space. The space is often referred to as latent space and may be seen as mathematically corresponding to a manifold that particularly preserves the relevant and discards the irrelevant information. Beyond this dimensionality reduction, "more appropriate space" may also mean that similar information is better clustered in that space and can be better told apart. Encoders which typically perform the transformation into the embedding space can: be learnt based on the task, can be taken from a generic pre-trained encoder and possibly refined, or can be manually constructed, for example. An example of a generic text encoder known in the art is Contrastive Language-Image Pre-training, CLIP. In addition to text, images can also be embedded into different space, i.e. a more appropriate and condensed space in a similar manner. As this yields a more compact representation of the relevant information (e.g. memory footprint), it allows for reducing computational overhead and is, thus, particularly useful for models such as latent diffusion models.

Conditioning: Conditioning may refer to taking a piece of information in some representation and making a processing step output (i.e. the output of a processing step that processes input information) depend on it. Technically conditioning can be achieved by an attention mechanism, generally known for neural networks. In order to generate its output the generative model processes some input (which may even be a random or constant input). To guide the sequence of processing steps to fulfil certain conditions at the output (e.g., a heart must be located at this position), information can be injected into these processing steps, which influences the way these processing steps operate on their inputs. In the present disclosure, the anatomical map (e.g. map of semantic anatomical regions) is used for conditioning. In one example, the anatomical map may be taken as is to influence the operations in the model, e.g. by adaptive normalization, where the intermediate data representations within the model are normalized (e.g. on voxel level) by parameters computed from this map (e.g. from information found at the corresponding location in the map). In another example, not the original representation of the data (e.g. text or images), such as the anatomical map, is used, but a representation in another space (embeddings, cf. above). Processing steps in the model can attend to these embeddings to process and recombine their inputs in a manner that is influenced by the information. The above may be referred to as an attention mechanism. An example for this attention mechanism is multi-head cross attention.

Generally, attention means input information in a processing step is influenced (also referred to as impacted or modulated) by another piece of information the model attends to. This influence can be multiplicative or additive. For example, the model may be in the process of generation of an artificial image and at layer i may represent each location by a feature vector of information ("query"). On the other hand the model may have processed the anatomical map to also represent each location by a set of vectors of information (e.g. after applying a learnt linear projection to the anatomical map before) ("keys" and "value").

By attention the information may be fused and propagated to the next layer.

As mentioned above, multi-head cross attention may be used, wherein "cross" refers to attending to some other piece of information (e.g. artificial image to anatomical map as opposed to "self" attention e.g. artificial image attends to itself). "Multi-head" refers to multiple of these attention blocks that co-exist at the same place but have different learnt parameters. In contrast to spatial convolutions, certain attention mechanisms are known to better model long-range dependencies.

The above-described influence or impact on the processing steps within the model may be referred to as conditioning.

It is noted that an advantage of using embeddings is that different sources of information (e.g. text or images) may be embedded into the same space, so that the original representation is less relevant in the course of processing steps.

The features and advantages outlined in the context of the method similarly apply to the system, computer program product, and computer-readable medium.

### BRIEF DESCRIPTION OF THE DRAWINGS

Fig 1 illustrates a method for creating a synthetic patient model according to the present disclosure,
Fig. 2 illustrates a system according to the present disclosure.
Fig. 3 illustrates exemplary inputs and outputs of a method according to the present disclosure.
Fig. 4 illustrates an exemplary workflow according to the present disclosure.

### DETAILED DESCRIPTION OF EMBODIMENTS

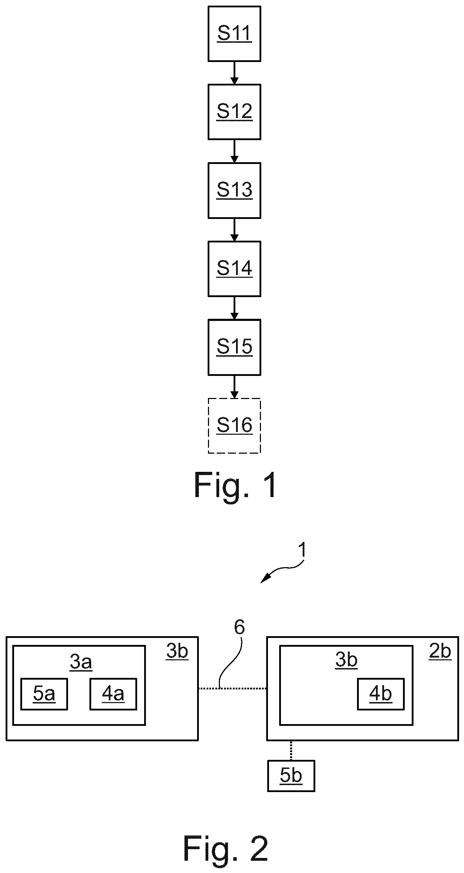

Fig 1 illustrates a computer-implemented method for creating a synthetic patient model comprising a synthetic patient image.

The method comprises, in step S 11, obtaining a data set comprising medical information The medical information comprises an anatomical description comprising at least information specifying an anatomical structure to be depicted in the synthetic patient image, and imaging information specifying image formation characteristics of the synthetic patient image, and wherein the medical information optionally comprises anamnesis and physical examination descriptions. Particularly, at least part of the medical information may be textual. An example for medical information according to the present disclosure is a medical report. The medical report may be retrieved from data storage or generated based on a user input, directly or via a language model, wherein the user provides a prompt. Medical reports generally have a structured form, which simplifies further processing thereof.

The method comprises, in step S12, generating an anatomical map of the anatomical structure by means of anatomy modelling using the anatomical description as an input. As an example, the anatomical map may be a labelled map, particularly a semantic label map.

The method comprises, in step S13, conditioning a generative model using the anatomical map. The generative model may be an image generative model that outputs a synthetic patient image, such as a diffusion model, like a latent diffusion model. As such, step S 13 may particularly provide conditioning that leads to anatomically appropriate shapes and arrangements being output in the synthetical medical image.

The method comprises, in step S14, embedding a subset of the medical information, the subset comprising textual information and including at least the imaging information, particularly in the form of textual information, in the generative model. As an example, the embedded information may comprise information on image formation characteristics, such as an image modality, to be modelled by the generative model, such that, for example, the synthetic patient image is given proper intensities (e.g. Hounsfield Units in CT) and/or contrast properties.

In one illustrative example, where step S12 yields 3D information and the modality to be created for the synthetic patient model is a specified projection, the generative model can learn this projection, have it explicitly incorporated as a non-learnable intermediate part of the architecture, or, for example, it can just learn to generate CT-like images of photon absorption per voxel, so that a classical forward projector (e.g. a C-arm simulator) can be applied thereafter to carry out the projection step.

The method comprises, in step S15, creating the synthetic patient model including a synthetic patient image by means of the generative model. To that end, the generative model ingests the provided information, and its outputs are included in the synthetic patient model. Particularly, the generative model outputs the synthetic patient image which is included in the synthetic patient model.

In optional step S 16, one or more tools that are based on one or more synthetic patient models obtained by the preceding steps may be provided.

The one or more tools may comprise an analysis support tool for analysis support for a medical practitioner, particularly for supporting medical image analysis.

Alternatively or in addition, the one or more tools may comprise a training tool for training of a student and/or medical practitioner, for training accuracy of medical image analysis.

Alternatively or in addition, the one or more tools may comprise a training data generation tool configured to generate training data for a machine learning model, the training data based on and/or derived from the one or more synthetic patient models, particularly for providing a trained machine learning model that analyzes medical images.

Fig. 2 illustrates a schematic, not-to scale view of a system 1 according to the present disclosure.

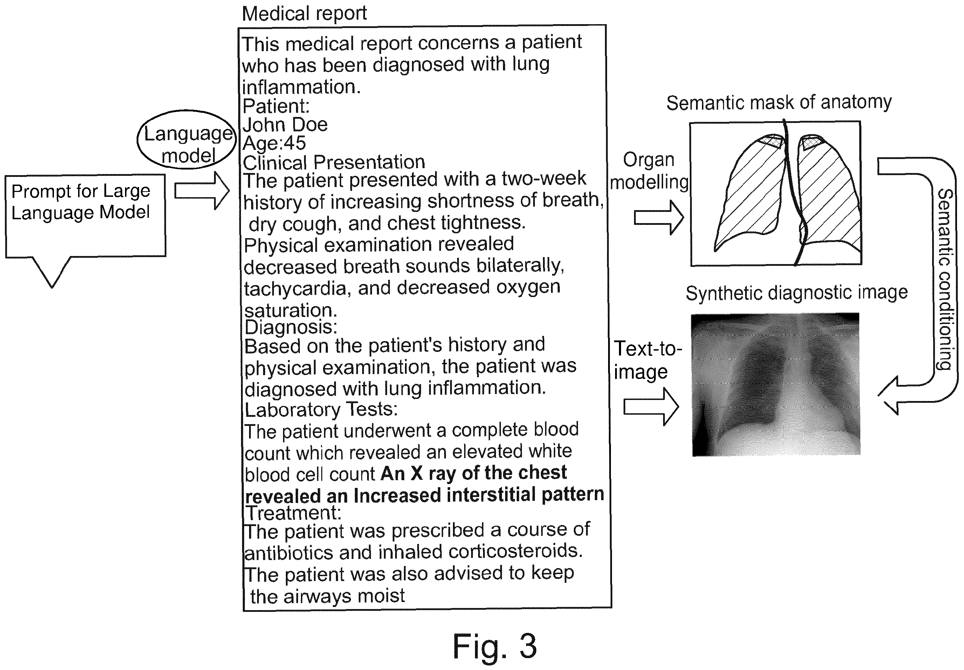

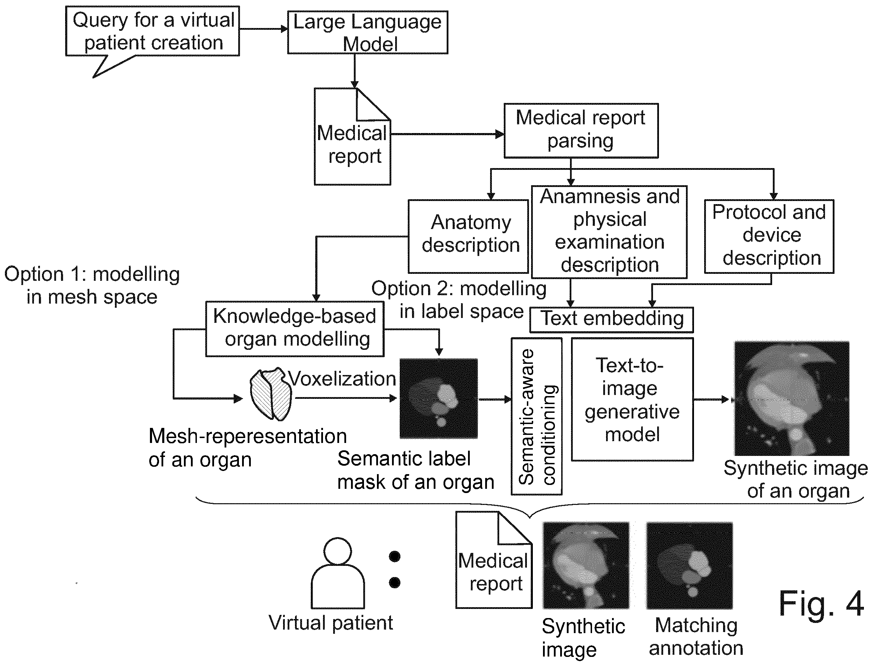

The system is configured to carry out the method of the present disclosure, e.g. as described above in the context of Fig. 1 or below in the context of Figs. 3 and 4.

Specifically, the system comprises one or more processing devices configured to carry out the method of the present disclosure.

As an example, the system as shown in Fig. 2 comprises first and second processing devices 2a and 2b connected via a data connection. This allows for distributed execution of the method of the present disclosure Alternatively, the method may be carried out ventrally on a single processing device 2a or 2b, in which case the other processing device and the data connection may be omitted.

In the present example, a first and second display devices 3a and 3b are illustrated, as an example, one of the display devices may be used in the step of obtaining at least part of the medical data, for example by visualizing for the user the content of the medical data. The other display device may be used for outputting a visualization of the synthetic patient model. Alternatively, a single display device may be used in the step of obtaining at least part of the medical data and for outputting the visualization of the synthetic patient model. In this case, one of the display devices may be omitted.

One or both display devices may be connected to at least one of, in particular both of the illustrated processing devices via data connections 6. Alternatively or in addition, one or both display devices may, respectively, be integrally formed with one of the processing devices.

A first and second user interface 4a and 4b may be provided, for example via a respective display device displaying a user interface. For example, one of the user interfaces may be used in the step of obtaining at least part of the medical data, for example by allowing a user to view, input, modify, and/or select data to be included in the medical data. The other user interface may be used for a user to view and/or interact with a visualization of the synthetic patient model. Alternatively, a single user interface may be used in the step of obtaining at least part of the medical data and for user to view and/or interact with a visualization of the synthetic patient model. In this case, one of the user interfaces may be omitted.

A first and a second input device 5a and 5b may be provided. The input devices may allow for the user to interact with the processing device, particularly with the user interface. In particular, the input devices may allow for a user to input, modify and/or select data, e.g. the structure medical data, and/or view and/or interact with a visualization of the synthetic patient model. Input devices may comprise a mouse and/or a keyboard and/or a touch display, which may be integrally formed with the display device(s) 3a and 3b.

The present invention also provides a computer program product comprising computer-readable instructions which, when executed by a computer, cause the computer to carry out the method of the present disclosure, e.g. as described above in the context of Fig. 1 or below in the context of Figs. 3 or 4

The present invention also provides a computer readable medium having stored thereon computer-readable instructions which, when executed by a computer, cause the computer to carry out the method of the present disclosure, e.g. as described above in the context of Fig. 1 or below in the context of Figs. 3 or 4.

In the following, more aspects and advantages of the method of the present disclosure will be provided.

As explained above, having exhaustive information about a patient covering all aspects of a pathological state opens a prospective to detailed data analysis. Additionally, the ability to tweak some aspects of a pathology in a patient and immediately see how it can affect the sections of a medical report and diagnostic images can be a useful tool for a wide range of end-users. However, complete unified medical records containing all required information accompanied by and including all sorts of desired diagnostic images can hardly be acquired under real-world conditions. The present disclosure aids in overcoming such challenges.

Understanding correlations between pathological states, symptoms, and depiction of a pathology in a diagnostic image can be difficult. As an example, demonstration of transition of disease manifestation with varying input conditions can be beneficial for educational purposes, e.g., for medical students. As another example, having a contrastive example can help accepting or declining a diagnostic hypothesis. Hence, a clinician can benefit from using an adjustable virtual patient by modelling different pathological conditions and understanding how the pathological condition is manifested.

In addition, the proposed method of virtual patient modelling helps to overcome the lack of data. The method can be used for generating (complete) multi-modal datasets, containing medical reports with textual description of a virtual patient and a disease, accompanying diagnostic image, and accurately matching annotation.

The generated multi-modal dataset can be used for data augmentation to train a machine learning algorithm for solving classification or segmentation task.

The method and system of the present disclosure allow for virtual patient modelling. The method of the present disclosure allows to generate a patient dataset containing a medical report, one or multiple respective diagnostic image(s) of a desired modality, and matching ground truth annotation(s). The method of the present disclosure may make use knowledge-driven organ modelling and a synthesis using generative neural networks conditioned on semantic anatomical information. The method may also make use of a large language model, e.g. for processing user prompts to generate medical information. The method can be used as support tool. For example, it may be used as an assisting tool to support clinicians in contrastive medical example simulations, as an educational tool for e.g., medical students or professionals in training, or as a data augmentation approach for subsequent machine learning based algorithmic training.

Making reference to the figures, the method and system of the present disclosure may make use of at least some of the components illustrated in Fig. 3. The components may be seen as being components of a system for virtual patient modelling.

Component 1 is a large language model that generates medical reports according to the provided prompts. The prompt may include either high-level description of a patient ("Mid-aged male patient diagnosed with tumor in basal abdomen") or a more detailed anamnesis with symptomatic descriptions ("Male patient, X years old, diagnosed with a medium sized tumor of Y cm in basal abdomen, suffers from a set of Z chronic diseases, etc."). This component is very suitable for obtaining medical information for being ingested by the subsequent modelling stages. However, it is optional. There are other methods that are equally conceivable for obtaining such medical information.

Component 2 is an organ modelling module that models organs and uses anatomical characteristics provided in the generated medical report along with morphological information about a lesion (e.g., "tumor of Y cm in basal abdomen") to create an anatomical atlas, e.g., in form of a mesh representation or a label mask of the target organ. Additionally, medical report information can be used to define a set of rules or model parameters aiming at a knowledge-based description of plausible organ/patient physiological behavior (e.g., a particular organ motion, or an illustration of possible posttreatment scenarios).

Component 3 is a generative neural network with semantic-aware conditioning, which uses textual description of a pathology and imaging device protocol as well as an anatomical label mask to synthesize a diagnostic image. It is noted that alternatively or in addition to a synthetic diagnostic image, the method of the present disclosure may also comprise generating synthetic interventional images as part of the synthetic patient model.

As an example, as illustrated in Fig. 3, the prompt containing query and initial information about the desired characteristics of a virtual patient may be passed to the large language model. The generated medical report may be used for organ modelling to create a semantic mesh or mask of anatomies. Third, the anatomy mask is used as a semantic conditioning of the generative model, which combines diagnosis information, imaging device protocol and anatomical information to synthesize a diagnostic image.

An exemplary method according to the present disclosure is described below making reference to Fig. 4. The proposed method of virtual patient modelling is based on medical report, respective diagnostic images, and matching ground truth generation. In Fig. 4, a flowchart illustrates that a medical report, generated by a large language model according to a query, is parsed into anatomy, anamnesis and physical examination description, and imaging device protocol descriptions. The anatomical description contains parameters used in the knowledge-based organ modelling to construct a mesh (option 1) or label (option 2) representation of a target organ. The mesh or label representation is subsequently used as an input condition in the generative model to control the structural characteristics of a generated image. Anamnesis, physical examination results and device protocol descriptions are embedded in the model to define the textural characteristics. As a result, the method allows to create a virtual patient representation that contains a medical report, respective diagnostic image, and matching ground truth annotation.

More specifically, the example shown in Fig. 4 may be carried out, in the following exemplary manner.

A user may query the large language model to generate a medical report of a patient with specific pathological states. Additional information on the desired anamnesis can be provided within the prompt. The large language model can be a GPT-like architecture trained on a set of medical reports or an unsupervised language model fine-tuned on medical domain data.

The generated medical report is parsed into descriptions of anatomy, anamnesis and physical examination, imaging modality, imaging device, and protocol. The anatomy information is used within organ modelling to create an atlas of the anatomy, e.g., in form of a mesh (option 1) or label mask (option 2) representation. The organ modelling can e.g., be performed with a knowledge-based approach.

The label mask is passed to the generative model that is semantically conditioned on the anatomy. At the same time, anamnesis and physical examination information, imaging modality type, protocol parameters are embedded within the generative model to condition the appearance of the generated anatomical structures. The embedding and conditioning are performed via the latent space, and the generative model follows the latent diffusion model approach.

Optionally, the proposed methods of virtual patient modelling can also be used to generate 1-D data, for example ECG. In this case, the above-described methods may additionally include a text-to-signal generative model, that is conditioned on text embeddings containing general symptomatic description, anamnesis and other information contained in the synthetically generated medical report. The method and system of the present disclosure, for example as explained above, may have different applications.

One application is the use as a data augmentation tool. Such a tool may provide an exhaustive and complete set of data, e.g., including textual description, images, ground truth annotations. As such the data yielded by the method and system of the present disclosure may, for example, be used for training machine learning models, e.g., machine learning models used as diagnostic or predictive tools.

The method of the present disclosure may be used as a support tool for medical professionals in training and/or in execution of their tasks.

The support tool may be an educational tool for e.g., medical students or professionals in training. The modelling system allows the medical students or professionals in training to simulate pathological conditions via altering anamnesis and patients' complaints.

The support tool may be an assistant tool, e.g. for a clinician, for constructing contrastive examples. The contrastive examples aim at supporting decision making, since they can be used to compare real patient's disease manifestation with simulated scenarios. As such, the tool may be a support tool in analyzing and diagnosis.

In the following, more detailed examples are provided that can be incorporated in and combined with any of the methods and systems of the present disclosure, particularly in the ones outlined above.

Specifically, knowledge-based modelling of anatomy, such as organs, will be described below in an exemplary fashion.

The input for the modelling may be an anatomy description, e.g. from structured text data or other diagnostic images. Such input may comprise:
Type of the target organ(s), e.g. heart, lung, liver, kidney, etc.

General or pathological shape descriptors, such as:
For the heart:
cardiomyopathy type characterizing hypertrophy (thickened myocardium), apical hypoplasia, truncated, enlarged, elongated, spherical chambers (LV, RV, RA, LA), one chamber wrapped around the other, chamber dilation, "banana-shaped" RV etc.

Other pathological conditions influencing the anatomy, such as calcified valves
Physiological states the organ can be in, e.g.:
cardiac phase, e.g. end systole (ES) or end diastole (ED)
inhale or exhale phase for the lung

Location and characteristics of known lesions, e.g. lung nodules, liver lesions, etc.

Previous therapy impacts, e.g. heart valve clips, artificial or implanted protheses (e.g. valves), stents, etc.

Indirect descriptors, e.g., age, BMI, ejection fraction for the heart etc.

Modelling may be carried out as described below, for example by (1) modelling organs using surface meshes or (2) volumetric models (e.g., tetrahedral meshes or voxel masks).

Each subpart of the model can have semantic labels (e.g. for the heart: RA, LA, LV, RV, valve XY, calcification or the like), which attach information to surfaces, volumes or locations.

Optionally image formation specifics may be provided. For example, if the targeted imaging modality is a 2D slice or projection, the 3D model needs to be translated into that space at the end before (or after if the modality allows, e.g. CTA to Xray) the image-specific texture/intensities are generated on top of the anatomy ("text-to-image generative model").

The modelling may be carried out as follows.

Organs may be described by average representations obtained e.g. from segmenting, registering and averaging a set of arbitrary patient images or from known available human phantoms (e.g. the XCAT phantom). This can serve as an atlas of the anatomy.

This approach can be extended by more elaborated statistical models, i.e. beyond mean model, e.g. by component analysis (PCA, ICA etc. or difference modes between two states, e.g. healthy and pathology XY).

These statistical models and corresponding component analyses yield statistical modes which can be weighted and added (e.g. linearly) to the average model. This customizes the average model to approach a patient specific anatomy.

These statistical modes may also include organ states such as inhale/exhale phases for the lung or the cardiac phase of the heart (connected to contraction or torsion geometric features), which can, for example, be modelled as a spatial difference mode between organ state A and organ state B.

Shape variations of the anatomy (e.g. an organ, sub-part of an organ or a constellation of organs) can also be described as volumetric (regular grid or scattered) vector deformation fields, e.g. modelled analytically or obtained from registering pair-wise or case-atlas-wise images or models.

Therapy devices can be modelled in a similar way or from CAD models available directly from the manufacturer and identified by a unique name, e.g. a valve prothesis.

Generally, the knowledge-based modelling translates (e.g. subjective) descriptions from text to a set of parameters governing the degrees of freedom of the different anatomical model components and/or how they are combined, e.g. by weighting of certain statistical modes on top of an average model. For this translation text attributes such as strong, mild, severe or locational descriptors (e.g. proximal, distal, anterior, posterior etc.) can be used.

The former can be refined by user interaction or from quantitative information, e.g. LV chamber volume, lesion diameter etc., or can be derived from another diagnostic image, for example from real diagnostic ultrasound images that have been acquired for the patient in case a synthetic and/or diagnostic CTA is the current modality of interest. To this other diagnostic image, e.g. an available previous diagnostic image, the model components can be fitted, e.g. using model-based segmentation, to tailor the anatomical model even further to the patient specific characteristics.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

### LIST OF REFERENCE SIGNS:

Steps S11 to S16
System 1
Processing device 2a, 2b
Display device 3a, 3b
User interface 4a, 4b
Input device 5a, 5b
Data connection 6

## Claims

1. A computer-implemented method for creating a synthetic patient model comprising a synthetic patient image, the method comprising
obtaining (S11) a data set comprising medical information,
wherein the medical information comprises an anatomical description comprising at least information specifying an anatomical structure to be depicted in the synthetic patient image, and imaging information specifying image formation characteristics of the synthetic patient image, and wherein the medical information optionally comprises anamnesis and physical examination descriptions;
generating (S12) an anatomical map of the anatomical structure by means of anatomy modelling using the anatomical description as an input;
conditioning (S13) a generative model using the anatomical map;
embedding (S14) a subset of the medical information, the subset comprising textual information and including at least the imaging information, in the generative model; and
creating (S 15) the synthetic patient model including a synthetic patient image by means of the generative model.

2. The method of claim 1, wherein conditioning the generative model using the anatomical map comprises conditioning the generative model directly with the anatomical map or generating an embedding of the anatomical map and conditioning the generative model with the embedding of the anatomical map.

3. The method of claim 1 or 2,
wherein generating the anatomical map comprises obtaining spatial information of the anatomical structure by means of the anatomy modelling and generating a labelled mesh representation of the surface of the anatomical structure or a label mask of the anatomical structure.

4. The method of any of the preceding claims,
wherein the anatomical description comprises at least one of:
one or more organs to be modelled, such as heart, lung, liver, kidney,
one or more shape descriptors for the anatomical structure, particularly general shape descriptors and/or pathological shape descriptors, the shape descriptors optionally comprising at least one of: pathological conditions, particularly pathological conditions that influence anatomy, a pathology-specific shape and/or deformation of an anatomical structure, such as pathology-specific deformations of an anatomical structure, a physiological state of an organ to be modelled, such as cardiac phase or respiratory phase, a location and/or characteristic of a lesion to be modelled,
a previous medical intervention, such as a heart valve clip, a prosthesis, and/or a stent,
patient characteristics such as age, size, weight, BMI, ejection fraction for the hear; and/or
wherein the anatomical description is in the form of textual data and/or image data.

5. The method of any of the preceding claims,
wherein anatomy modelling comprises generating a surface model of the anatomical structure's surface using a surface mesh or generating a volumetric model of the anatomical structure,
wherein generating the anatomical map comprises generating labels for one or more portions of the surface model or the volumetric model, particularly labels associating information with the one or more portions, and
wherein the one or more portions comprise at least one of: surface portions, volumetric portions, locations,
in particular, wherein the modelling is a knowledge-based modelling making use of anatomical atlas data and optionally spatial data representative of artificial structures, such as prosthesis, heart valve clips, stents or the like.

6. The method of any of the preceding claims, wherein obtaining the data set comprising medical information comprises parsing information, for example, parsing textual information, and extracting, from the parsed information, information to be input into the anatomy modelling and/or to be embedded in the generative model.

7. The method of any of the preceding claims,
wherein obtaining the data set comprising medical information comprises:
receiving a user input specifying information to be included in the medical information and/or receiving user input from which information to be included in the medical information is derived, and
including the specified and/or derived information in the medical information; and/or
wherein at least part of the medical information is textual information; and/or
wherein the medical information comprises information in the form of a medical report.

8. The method of any of the preceding claims,
wherein the user input comprises a selection of information and the selected information is included in the medical information, and/or
wherein the user input comprises formalized free input, for example a user filling in a digital form, and the method comprises extracting the medical information by processing the formalized free input, and/or
wherein the user input comprises one or more prompts for a language model, particularly a large language model such as a Generative Pre-trained Transformer, GPT,-based model, and the method comprises providing the one or more prompt to the language model and the language model outputting the medical information by processing the one or more prompts.

9. The method of any of the preceding claims wherein the embedding of the subset of the medical information in the generative model comprises transforming the subset of medical information into an embedding space, particularly comprising tokenization of textual information comprised in the subset of the medical information and generating a condensed representation by transforming the tokens with a, particularly given or learned, transformation.

10. The method of any of the preceding claims, the method further comprising, as part of creating the synthetic patient model, generating synthetic 1D data, such as signals like ECG or EEG signals, by means of a text-to-signal generative model conditioned on one or more text embeddings of textual information comprised in the medical information.

11. The method of any of the preceding claims,
wherein the generative model is a machine learning model, particularly a diffusion model, particularly a latent diffusion model, and/or
the synthetic patient model further comprises:
a synthetic medical report, particularly comprising at least part of the medical information, and/or
an output of the anatomy modelling, such as the anatomical map, and/or
a/the synthetic 1D data.

12. The method of any of the preceding claims, comprising providing (S16) one or more tools based on one or more synthetic patient models obtained by the method of any of claims 1 to 11, the one or more tools comprising
an analysis support tool for analysis support for a medical practitioner, particularly for supporting medical image analysis, and/or
a training tool for training of a student and/or medical practitioner, for training accuracy of medical image analysis, and/or
a training data generation tool configured to generate training data for a machine learning model, the training data based on and/or derived from the one or more synthetic patient models, particularly for providing a trained machine learning model that analyzes medical images.

13. A system (1) comprising a processing device configured to carry out the method of any of the preceding claims.

14. A computer program product comprising computer-readable instructions which, when executed by a computer, cause the computer to carry out the method of any of claims 1 to 10.

15. A computer-readable medium having stored thereon computer-readable instructions which, when executed by a computer, cause the computer to carry out the method of any of claims 1 to 10.
